(19) Europäisches Patentamt
European Patent Office
Office européen des brevets

(11) Veröffentlichungsnummer: **0 237 908**
**B1**

(12) **EUROPÄISCHE PATENTSCHRIFT**

(45) Veröffentlichungstag der Patentschrift: **29.08.90**

(51) Int. Cl.[5]: **C07D 401/06**, C07D 401/14, C07D 471/10, A61K 31/44

(21) Anmeldenummer: **87103367.6**

(22) Anmeldetag: **09.03.87**

(54) **Pyridinderivate.**

(30) Priorität: **19.03.86 DE 3609142**

(43) Veröffentlichungstag der Anmeldung: **23.09.87 Patentblatt 87/39**

(45) Bekanntmachung des Hinweises auf die Patenterteilung: **29.08.90 Patentblatt 90/35**

(84) Benannte Vertragsstaaten: **AT BE CH DE ES FR GB IT LI NL SE**

(56) Entgegenhaltungen:
**EP-A- 0 180 794**
**DE-A- 1 695 694**

(73) Patentinhaber: **MERCK PATENT GESELLSCHAFT MIT BESCHRÄNKTER HAFTUNG, Frankfurter Strasse 250 Postfach 4119, D-6100 Darmstadt(DE)**

(72) Erfinder: **Böttcher, Henning, Dr., Soderstrasse 95, D-6100 Darmstadt(DE)**
Erfinder: **Seyfried, Christoph, Dr., Mathildenstrasse 6, D-6104 Seeheim-Jugenheim(DE)**
Erfinder: **Minck, Klaus-Otto, Dr., Büchestrasse 5, D-6105 Ober-Ramstadt(DE)**

**EP 0 237 908 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

**Beschreibung**

Die Erfindung betrifft neue Pyridinderivate der Formel I

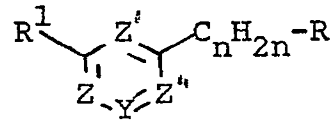

worin eine der Gruppen Y, Z, Z' und Z" N, die anderen dieser Gruppen N, jeweils CH, 1, 2 oder 3,

R 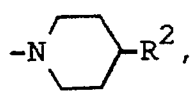, 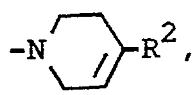, 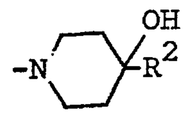,

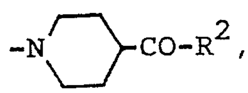, 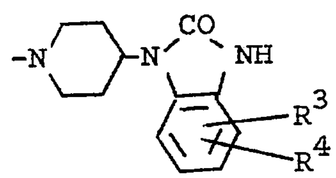.

oder 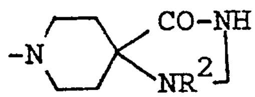,

$R^1$ u. $R^2$ jeweils unsubstituierte oder ein- oder zweifach durch Alkyl und/oder Alkoxy mit jeweils 1-4 C-Atomen, F, Cl, Br, OH und/oder $CF_3$ substituierte Phenyl- oder 2- oder 3-Thienylreste und

$R^3$ u. $R^4$ jeweils H, Alkyl oder Alkoxy mit jeweils 1-4 C-Atomen, F, Cl, Br, OH oder $CF_3$ bedeuten, wobei jedoch Y CH bedeutet, wenn

R 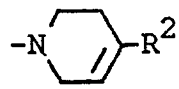

bedeutet, sowie ihre Salze.

Der Erfindung lag die Aufgabe zugrunde, neue Verbindungen aufzufinden, die zur Herstellung von Arzneimitteln verwendet werden können.

Es wurde gefunden, daß die genannten Substanzen bei guter Verträglichkeit wertvolle pharmakologische Eigenschaften besitzen. So zeigen sie z.B. das Zentralnervensystem beeinflussende, vorzugsweise dampfende (z.B. sedierende, tranquillierende, neuroleptische und/oder antidepressive) Wirkungen. Im einzelnen haben die Verbindungen eine dämpfende Wirkung auf das Verhalten bei Mäusen (Methodik vgl. Irwin, Psychopharmacologia 13 (1968), 222-257), hemmen bei Mäusen das durch Apomorphin induzierte Kletterverhalten (Methodik vgl. Costall et al., European J. Pharmacol. 50 (1968), 39-50) oder induzieren contralaterales Drehverhalten in Hemiparkinson-Ratten (feststellbar nach der Methode von Ungerstedt et al., Brain Res. 24 (1970), 485-493), ohne daß nennenswerte kataleptische Nebenwirkungen auftreten (Methodik vgl. Dolini-Stola, Pharmakopsychiat. 6 (1973), 189-197). Weiterhin hemmen die Substanzen die Bindung von tritiierten Dopaminagonisten und -antagonisten an striäre Rezeptoren (feststellbar nach der Methode von Schwarcz et al., J. Neurochemistry 34 (1980), 772-778, und Creese et al., European J.Pharmacol. 46 (1977), 377-381). Zusätzlich hemmen die Verbindungen den Zungen-Kieferreflex bei der narkotisierten Ratte (feststellbar in Anlehnung an die Methoden von Barnett et al., European J.Pharmacol. 21 (1973), 178-182, und von Ilhan et al., European J.Pharmacol. 33 (1975), 61-64). Weiterhin treten analgetische und blutdrucksenkende Wirkungen auf; so wird der bei kathetertragenden wachen, spontan hypertonen Ratten (Stamm SHR/NIH-MO//CHB-EMD; Methode vgl. Weeks und Jones, Proc.Soc.Exptl.Biol.Med. 104 (1960), 646-648) direkt gemessene arterielle Blutdruck nach intragastraler Gabe der Verbindungen gesenkt.

Verbindungen der Formel I und ihre physiologisch unbedenklichen Säureadditionssalze können daher als Arzneimittelwirkstoffe und auch als Zwischenprodukte zur Herstellung anderer Arzneimittelwirkstoffe verwendet werden.

Gegenstand der Erfindung sind die Pyridinderivate der Formel I sowie ihre Salze.

Gegenstand der Erfindung ist ferner ein Verfahren zur Herstellung von Pyridinderivaten der Formel I sowie von deren Salzen, dadurch gekennzeichnet, daß man eine Verbindung der Formel II

Py-A-$X^1$ II

worin

Py den Rest 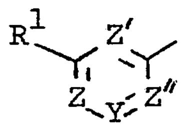

A die Gruppe $-C_nH_{2n}-$,

$X^1$ X oder $NH_2$,

X Cl, Br, J, OH oder eine reaktionsfähig funktionell abgewandelte OH-Gruppe bedeuten und

$R^1$, Y, Z, Z', Z" und n die angegebenen Bedeutungen haben,

mit einer Verbindung der Formel III

$X^2-CH_2CH_2-G-CH_2-X^3$ III

worin

$X^2$ u. $X^3$ gleich oder verschieden sind und, falls $X^1 = NH_2$ ist, jeweils X, andernfalls zusammen NH und

G $-CHR^2-CH_2-$, $-CR^2=CH-$, $-CR^2(OH)-CH_2-$,

$-CH(CO-R^2)-CH_2-$, 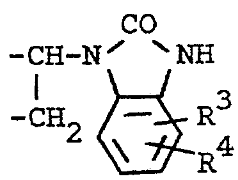 oder

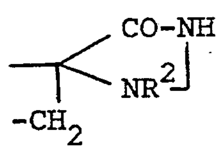

bedeuten und

$R^2$, $R^3$ und $R^4$ die angegebenen Bedeutungen haben,

umsetzt

oder daß man eine sonst der Formel I entsprechende Verbindung, die jedoch an Stelle eines oder mehrerer Wasserstoffatome eine oder mehrere reduzierbare Gruppen und/oder eine oder mehrere zusätzliche C-C- und/oder C-N-Bindungen enthält, mit einem reduzierenden Mittel behandelt

oder daß man zur Herstellung einer Verbindung der Formel I, worin

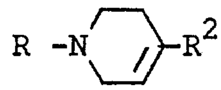

ist, eine Verbindung der Formel IV

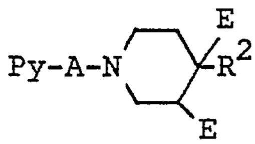

IV

worin

der eine Rest E X, CN oder $NH_2$,
der andere Rest E H bedeutet und
Py, A, $R^2$ und X die angegebenen Bedeutungen haben

mit einem HE-abspaltenden Mittel behandelt

und/oder daß man gegebenenfalls in einer Verbindung der Formel I eine O-Alkylgruppe unter Bildung einer OH-Gruppe spaltet

und/oder daß man eine Base der Formel I durch Behandeln mit einer Säure in eines ihrer Salze umwandelt.

Die Verbindungen der Formel I umschließen die 2-$R^1$-6-RA-pyridine (I'), die 2-$R^1$-4-RA-pyridine (I''), die 3-$R^1$-5-RA-pyridine (I''') und die 2-RA-4-$R^1$-Pyridine (I''''). Bevorzugt sind I''' und I''''.

Im einzelnen seien besonders erwähnt:

(4-$R^2$-piperidinoalkyl)-pyridine (Ia), nämlich

2-$R^1$-6-(4-$R^2$-piperidinoalkyl)-pyridine(Iaa)
2-$R^1$-4-(4-$R^2$-piperidinoalkyl)-pyridine(Iab)
3-$R^1$-5-(4-$R^2$-piperidinoalkyl)-pyridine(Iac)
2-(4-$R^2$-piperidinoalkyl)-4-$R^1$-pyridine(Iad);

(4-$R^2$-1,2,3,6-tetrahydropyridyl-alkyl)-pyridine (Ib), nämlich

2-$R^1$-6-(4-$R^2$-1,2,3,6-tetrahydropyridyl-alkyl)-pyridine(Iba)
2-$R^1$-4-(4-$R^2$-1,2,3,6-tetrahydropyridyl-alkyl)-pyridine(Ibb)
2-(4-$R^2$-1,2,3,6-tetrahydropyridyl-alkyl)-4-$R^1$-pyridine(Ibd);

(4-$R^2$-4-hydroxypiperidinoalkyl)-pyridine (Ic), nämlich

2-$R^1$-6-(4-$R^2$-4-hydroxypiperidinoalkyl)-pyridine(Ica)
2-$R^1$-4-(4-$R^2$-4-hydroxypiperidinoalkyl)-pyridine(Icb)
3-$R^1$-5-(4-$R^2$-4-hydroxypiperidinoalkyl)-pyridine(Icc)
2-(4-$R^2$-4-hydroxypiperidinoalkyl)-4-$R^1$-pyridine(Icd);

(4-$R^2$-CO-piperidinoalkyl)-pyridine (Id), nämlich

2-$R^1$-6-(4-$R^2$-CO-piperidinoalkyl)-pyridine(Ida)
2-$R^1$-4-(4-$R^2$-CO-piperidinoalkyl)-pyridine(Idb)
3-$R^1$ 5-(4-$R^2$-CO-piperidinoalkyl)-pyridine(Idc)
2-(4-$R^2$-CO-piperidinoalkyl)-4-$R^1$-pyridine(Idd);

[4-(2-oxo-4,5,6 oder 7-$R^3$-4,5,6 oder 7-$R^4$-benzimidazolin-1-yl)-piperidinoalkyl]-pyridine (Ie), nämlich

2-$R^1$-6-[4-(2-oxo-4,5,6 oder 7-$R^3$-4,5,6 oder 7-$R^4$-benzimidazolin-1-yl)-piperidinoalkyl]-pyridine(Iea)
2-$R^1$-4-[2-(2-oxo-4,5,6 oder 7-$R^3$-4,5,6 oder 7-$R^4$-benzimidazolin-1-yl)-piperidinoalkyl]-pyridine(Ieb)
3-$R^1$-5-[4-(2-oxo-4,5,6 oder 7-$R^3$-4,5,6 oder 7-$R^4$-benzimidazolin-1-yl)-piperidinoalkyl]-pyridine(Iec)
2-[4-(2-oxo-4,5,6 oder 7-$R^3$-4.5,6 oder 7-$R^4$-benzimidazolin-1-yl)-piperidinoalkyl]-4-$R^1$-pyridine(Ied);
8-Pyridylalkyl-1-$R^2$-4-oxo-1,3,8-triazaspiro[4,5]decan-8-one (If), nämlich

8-(2-$R^1$-6-pyridylalkyl)-1-$R^2$-4-oxo-1,3,8-triazaspiro[4,5]decan-8-one(Ifa)
8-(2-$R^1$-4-pyridylalkyl)-1-$R^2$-4-oxo-1,3,8-triazaspiro[4,5]decan-8-one(Ifb)
8-(3-$R^1$-5-pyridylalkyl)-1-$R^2$-4-oxo-1,3,8-triazaspiro[4,5]decan-8-one(Ifc)
8-(4-$R^1$-2-pyridylalkyl)-1-$R^2$-4-oxo-1,3,8-triazaspiro[4,5]decan-8-one(Ifd).

Von diesen Gruppen von Verbindungen sind Ia, Ib und Ic bevorzugt, im einzelnen insbesondere Iac, Iba, Ibb, Ibd, Icc, weiterhin Iaa, Idc, Iec und Ifc.

In den Resten $R^1$ bis $R^4$ bedeutet Alkyl vorzugsweise Methyl, ferner auch Ethyl, n-Propyl, Isopropyl, n-Butyl, Isobutyl, sek.-Butyl oder tert.-Butyl. Alkoxy ist vorzugsweise Methoxy, ferner auch Ethoxy, n-Propoxy, Isopropoxy, n-Butoxy, Isobutoxy, sek.-Butoxy oder tert.-Butoxy.

Die Reste $R^1$ und $R^2$ sind bevorzugt unsubstituiertes Phenyl. Falls $R^1$ bzw. $R^2$ substituierte Phenylgruppen bedeuten, so sind diese vorzugsweise einfach substituiert. Sie können jedoch auch zweifach substituiert sein, wobei die Substituenten gleich oder verschieden sein können. Bevorzugte Substituenten an den Phenylgruppen sind Methyl, Ethyl, Methoxy, Ethoxy, F, Cl, Br und/oder OH. Im einzelnen sind $R^1$ und $R^2$ bevorzugt Phenyl, ferner o-, m- oder p-Tolyl, o-, m- oder p-Ethylphenyl, o-, m- oder p-Methoxyphenyl, o-, m- oder p-Ethoxyphenyl, o-, m- oder p-Fluorphenyl, o-, m- oder p-Chlorphenyl, o-, m- oder p-Bromphenyl, o-, m- oder p-Hydroxyphenyl, o-, m- oder p-Trifluormethylphenyl, 2,3-, 2,4-,2,5, 2,6-, 3,4- oder 3,5-Dimethylphenyl, 2,3-, 2,4-, 2,5-, 2,6-, 3,4- oder 3,5-Dimethoxyphenyl, 2,3-, 2,4-, 2,5-, 2,6-, 3,4- oder 3,5-Difluorphenyl, 2,3-, 2,4-, 2,5-, 2,6-, 3,4- oder 3,5-Dichlorphenyl, 2,3-, 2,4-, 2,5-, 2,6-, 3,4- oder 3,5-Dihydroxyphenyl, 2-Methyl-4-chlorphenyl, 2- oder 3-Thienyl, 5-Methyl-2-thienyl.

Die Reste $R^3$ und $R^4$ können gleich oder verschieden sein. Sie bedeuten vorzugsweise beide H. Weiterhin ist bevorzugt der eine dieser Reste H, der andere F, Cl, OH oder $CF_3$.

Der Parameter n ist vorzugsweise 1, die Gruppe $C_nH_{2n}$ (= "A") ist vorzugsweise $-CH_2-$, weiterhin vorzugsweise $-CH(CH_3)-$, $-(CH_2)_2$ oder $-(CH_2)_3-$.

Dementsprechend sind Gegenstand der Erfindung insbesondere diejenigen Verbindungen der Formeln I, I', I", I‴, I"" bzw. Ia bis Ifd, in denen mindestens einer der genannten Reste eine der vorstehend angegebenen, insbesondere der vorstehend angegebenen bevorzugten Bedeutungen hat. Einige bevorzugte Gruppen von Verbindungen entsprechen den vorstehend angegebenen Formeln, worin die nicht näher bezeichneten Reste und Parameter die bei Formel I angegebene Bedeutung haben, worin jedoch

(a) $R^1$ Phenyl, Tolyl, Methoxyphenyl, Fluorphenyl, Chlorphenyl, Hydroxyphenyl, Trifluormethylphenyl, Dimethoxyphenyl oder Thienyl bedeutet;
(b) $R^1$ Phenyl, o-, m- oder p-Tolyl, m- oder p-Methoxyphenyl, p-Fluorphenyl, p-Chlorphenyl, m- oder p-Hydroxyphenyl, m-Trifluormethylphenyl, 3,4-Dimethoxyphenyl oder 2-Thienyl bedeutet;
(c) $R^1$ Phenyl bedeutet;
(d) $R^2$Phenyl, Tolyl, Methoxyphenyl, Fluorphenyl, Chlorphenyl, Trifluormethylphenyl, Chlortrifluormethylphenyl oder Thienyl bedeutet;
(e) $R^2$ Phenyl, o-, m- oder p-Tolyl, p-Methoxyphenyl, p-Fluorphenyl, p-Chlorphenyl, m-Trifluormethylphenyl oder 2- oder 3-Thienyl bedeutet;
(f) $R^2$ Phenyl bedeutet;
(g) $C_nH_{2n}$-$CH_2$-, $-CH(CH_3)-$, $-(CH_2)_2-$ oder $-(CH_2)_3-$ bedeutet;
(h) $C_nH_{2n}$-$CH_2$- bedeutet;
(i) $R^1$ Phenyl, o-, m- oder p-Tolyl, m- oder p-Methoxyphenyl, p-Fluorphenyl, p-Chlorphenyl, m- oder p-Hydroxyphenyl, m-Trifluormethylphenyl, 3,4-Dimethoxyphenyl oder 2-Thienyl,
$R^2$Phenyl, o-, m- oder p-Tolyl, p-Methoxyphenyl, p-Fluorphenyl, p-Chlorphenyl, m-Trifluormethylphenyl oder 2- oder 3-Thienyl
und
$C_nH_{2n}$-$CH_2$-, $-CH(CH_3)-$, $-(CH_2)_2-$ oder $-(CH_2)_3-$ bedeuten;
(j) $R^1$ Phenyl, m- oder p-Methoxyphenyl, p-Fluorphenyl, p-Chlorphenyl, m-Hydroxyphenyl oder 3,4-Dimethoxyphenyl,
$R^2$Phenyl und
$C_nH_{2n}$-$CH_2$- bedeuten;
(k) $R^1$ und $R^2$ jeweils Phenyl, p-Fluorphenyl oder p-Chlorphenyl und
$C_nH_{2n}$-$CH_2$- bedeuten.

Einige Verbindungen der Formel I können ein oder mehrere asymmetrische Kohlenstoffatome besitzen. Sie können daher als Racemate, falls mehrere asymmetrische Kohlenstoffatome vorhanden sind, auch als Gemische mehrerer Racemate sowie in verschiedenen optisch-aktiven Formen vorliegen.

Die Herstellung der Verbindungen der Formel I erfolgt im übrigen nach an sich bekannten Methoden, wie sie in der Literatur (z.B. in den Standardwerken wie Houben-Weyl, Methoden der Organischen Chemie, Georg-Thieme-Verlag, Stuttgart; Organic Reactions, John Wiley & Sons, Inc., New York) beschrieben sind, und zwar unter Reaktionsbedingungen, wie sie für die genannten Umsetzungen bekannt und geeignet sind. Dabei kann man auch von an sich bekannten, hier nicht näher erwähnten Varianten Gebrauch machen.

Die Ausgangsstoffe für das beanspruchte Verfahren können gewünschtenfalls auch in situ gebildet werden, derart, daß man sie aus dem Reaktionsgemisch nicht isoliert, sondern sofort weiter zu den Verbindungen der Formel I umsetzt.

In den Verbindungen der Formel II ist $X^1$ vorzugsweise X; dementsprechend sind in den Verbindungen der Formel III $X^2$ und $X^3$ vorzugsweise zusammen NH. Der Rest X ist vorzugsweise Cl oder Br; er kann jedoch auch J, OH oder eine reaktionsfähig funktionell abgewandelte OH-Gruppe bedeuten, insbesondere Alkylsulfonyloxy mit 1-6 (z.B. Methansulfonyloxy) oder Arylsulfonyloxy mit 6-10 C-Atomen (z.B. Benzolsulfonyloxy, p-Toluolsulfonyloxy, 1- oder 2-Naphthalin-sulfonyloxy).

Dementsprechend sind die Verbindungen der Formel I insbesondere durch Umsetzung von Verbindungen der Formel Py-A-Cl oder Py-A-Br mit Verbindungen der Formel III, worin $X^2$ und $X^3$ zusammen eine NH-Gruppe bedeuten (nachstehend als IIIa bezeichnet), erhältlich.

Die Verbindungen der Formeln II und III sind zum Teil bekannt; die nicht bekannten Verbindungen der Formeln II und III können leicht analog zu den bekannten Verbindungen herstellt werden. Primäre Alkohole der Formel Py-A-OH sind z.B. durch Reduktion der entsprechenden Carbonsäuren oder ihrer Ester erhältlich. Behandeln mit Thionylchlorid, Bromwasserstoff, Phosphortribromid oder ähnlichen Halogenverbindungen liefert die entsprechenden Halogenide der Formel Py-A-Hal. Die entsprechenden Sulfonyloxyverbindungen sind erhältlich aus den Alkoholen Py-A-OH durch Umsetzung mit den entsprechenden Sulfonsäurechloriden. Die Jodverbindungen der Formel Py-A-J sind z.B. durch Einwirkung von Kaliumjodid auf die zugehörigen p-Toluolsulfonsäureester erhältlich. Die Amine der Formel Py-A-$NH_2$ sind z.B. aus den Halogeniden mit Phthalimidkalium oder durch Reduktion der entsprechenden Nitrile herstellbar.

Die Verbindungen der Formel IIIa sind teilweise bekannt (vgl. DE-OS 20 60 816) und z.B. erhältlich durch Umsetzung von 4-Piperidon mit metallorganischen Verbindungen der Formel M-$R^2$ (worin M ein Li-Atom oder MgHal bedeutet), anschließende Hydrolyse zu den entsprechenden 4-$R^2$-4-hydroxypiperidinen sowie, falls erwünscht, nachfolgende Dehydratisierung zu 4-$R^2$-3,4-dehydro-piperidinen und Hydrierung zu 4-$R^2$-piperidinen. Verbindungen der Formel III ($X^2$ und $X^3$ = jeweils X) sind z.B. herstellbar durch Reduktion von Diestern der Formel AlkylOOC-$CH_2$-G-COOAlkyl zu Diolen der Formel HO-$CH_2CH_2$-G-$CH_2OH$ (III, $X^2 = X^3 =$ OH) und gegebenenfalls anschließende Umsetzung mit $SOCl_2$ bzw. $PBr_3$.

Die Umsetzung der Verbindungen II und III verläuft nach Methoden, wie sie für die Alkylierung von Aminen aus der Literatur bekannt sind. Man kann ohne Gegenwart eines Lösungsmittels die Komponenten miteinander verschmelzen, gegebenenfalls im geschlossenen Rohr oder im Autoklaven. Es ist aber auch möglich, die Verbindungen in Gegenwart eines indifferenten Lösungsmittels umzusetzen. Als Lösungsmittel eignen sich z.B. Kohlenwasserstoffe, wie Benzol, Toluol, Xylol; Ketone wie Aceton, Butanon; Alkohole wie Methanol, Ethanol, Isopropanol, n-Butanol; Ether wie Tetrahydrofuran (THF) oder Dioxan; Amide wie Dimethylformamid (DMF) oder N-Methyl-pyrrolidon; Nitrile wie Acetonitril, gegebenenfalls auch Gemische dieser Lösungsmittel untereinander oder Gemische mit Wasser. Der Zusatz eines säurebindenden Mittels, beispielsweise eines Alkali- oder Erdalkalimetallhydroxids, -carbonats oder -bicarbonats oder eines anderen Salzes einer schwachen Säure der Alkali- oder Erdalkalimetalle, vorzugsweise des Kaliums, Natriums oder Calciums, oder der Zusatz einer organischen Base wie Triethylamin, Dimethylanilin, Pyridin oder Chinolin oder eines Überschusses der Aminkomponente Py-A-$NH_2$ bzw. der Verbindung der Formel IIIa kann günstig sein. Die Reaktionszeit liegt je nach den angewendeten Bedingungen zwischen einigen Minuten und 14 Tagen, die Reaktionstemperatur zwischen etwa 0 und 150°, normalerweise zwischen 20 und 130°.

Es ist ferner möglich, eine Verbindung der Formel I zu erhalten, indem man ein Vorprodukt, das an Stelle von Wasserstoffatomen eine oder mehrere reduzierbare Gruppe(n) und/oder eine oder mehrere zusätzliche C-C- und/oder C-N-Bindung(en) enthält, mit einem reduzierenden Mittel behandelt, vorzugsweise bei Temperaturen zwischen -80 und +250° in Gegenwart mindestens eines inerten Lösungsmittels.

Reduzierbare (durch Wasserstoff ersetzbare) Gruppen sind insbesondere Sauerstoff in einer Carbonylgruppe, Hydroxyl, Arylsulfonyloxy (z.B.p-Toluolsulfonyloxy), N-Benzolsulfonyl, N-Benzyl oder O-Benzyl.

Es ist grundsätzlich möglich, Verbindungen, die nur eine, oder solche, die nebeneinander zwei oder mehr dieser Gruppen bzw. zusätzlichen Bindungen enthalten, reduktiv in eine Verbindung der Formel I überzuführen. Vorzugsweise bedient man sich hierzu des nascierenden Wasserstoffs oder komplexer Metallhydride, ferner der Reduktion nach Wolff-Kishner.

Bevorzugte Ausgangsstoffe für die Reduktion entsprechen der Formel V

Py-L-R V

worin

L eine dem Rest A entsprechende Kette bedeutet, worin jedoch eine oder mehrere -$CH_2$-Gruppe(n) durch -CO-Gruppe(n) und/oder ein oder mehrere Wasserstoffatom(e) durch OH-Gruppe(n) ersetzt sind.

In den Verbindungen der Formel V ist L bevorzugt $-(CH_2)_{n-1}-CO-$ [im einzelnen -CO-, $-CH_2-CO-$ oder $-CH_2CH_2-CO-$], ferner z.B. $-CH(CH_3)-CO-$, $-COCH_2-$, -COCO-, $-COCH_2CO-$, $-CO-CH_2CH_2-$, $CH_2CO-CH_2-$, -CHOH-, $-CH_2-CHOH-$, $-(CH_2)_2-CHOH-$, $-CHOH-CH_2-$, -CHOH-CO-.

Verbindungen der Formel V sind z.B. herstellbar durch Umsetzung von IIIa mit einer Verbindung der Formel VI

Py-L-$X^1$ VI

worin

Py, L und $X^1$ die oben angegebenen Bedeutungen haben,

unter den Bedingungen, die oben für die Umsetzung von II und III angegeben sind.

Wird als Reduktionsmittel nascierender Wasserstoff verwendet, so kann man diesen z.B. durch Behandlung von Metallen mit schwachen Säuren oder mit Basen erzeugen. So kann man z.B. ein Gemisch von Zink mit Alkalilauge oder von Eisen mit Essigsäure verwenden. Geeignet ist auch die Verwendung von Natrium oder einem anderen Alkalimetall in einem Alkohol wie Ethanol, Isopropanol, Butanol, Amyl- oder Isoamylalkohol oder Phenol. Man kann ferner eine Aluminium-Nickel-Legierung in alkalisch-wäßriger Lösung, gegebenenfalls unter Zusatz von Ethanol verwenden. Auch Natrium- oder Aluminiumamalgam in wäßrig-alkoholischer oder wäßriger Lösung sind zur Erzeugung des nascierenden Wasserstoffs geeignet. Die Umsetzung kann auch in heterogener Phase durchgeführt werden, wobei man zweckmäßig eine wäßrige und eine Benzol- oder Toluol-Phase verwendet.

Als Reduktionsmittel können ferner besonders vorteilhaft komplexe Metallhydride, wie $LiAlH_4$, $NaBH_4$, Diisobutylaluminiumhydrid oder $NaAl(OCH_2CH_2OCH_3)_2H_2$ sowie Diboran eingesetzt werden, falls erwünscht unter Zusatz von Katalysatoren wie $BF_3$, $AlCl_3$ oder LiBr. Als Lösungsmittel eignen sich hierfür insbesondere Ether wie Diethylether, Di-n-butylether, THF, Dioxan, Diglyme oder 1,2-Dimethoxyethan sowie Kohlenwasserstoffe wie Benzol. Für eine Reduktion mit $NaBH_4$ sind in erster Linie Alkohole wie Methanol oder Ethanol, ferner Wasser sowie wäßrige Alkohole als Lösungsmittel geeignet. Nach diesen Methoden reduziert man vorzugsweise bei Temperaturen zwischen -80 und +150°, insbesondere zwischen etwa 0 und etwa 100°.

Besonders vorteilhaft lassen sich -CO-Gruppen in Säureamiden (z.B. solchen der Formel V, worin L $-(CH_2)_{n-1}-CO-$ bedeutet) mit $LiAlH_4$ in THF bei Temperaturen zwischen etwa 0 und 66° zu $CH_2$-Gruppen reduzieren.

Es ist ferner möglich, eine oder mehrere Carbonylgruppen nach der Methode von Wolff-Kishner zu $CH_2$-Gruppen zu reduzieren, z.B. durch Behandlung mit wasserfreiem Hydrazin in absolutem Ethanol unter Druck bei Temperaturen zwischen etwa 150 und 250°. Als Katalysator wird vorteilhaft Natriumalkoholat verwendet. Die Reduktion kann auch nach der Methode von Huang-Minlon variiert werden, indem man mit Hydrazinhydrat in einem hochsiedenden, mit Wasser mischbaren Lösungsmittel, wie Diethylenglykol oder Triethylenglykol, in Gegenwart von Alkali, wie Natriumhydroxid, umsetzt. Das Reaktionsgemisch wird in der Regel etwa 3-4 Stunden gekocht. Anschließend wird das Wasser abdestilliert und das gebildete Hydrazon bei Temperaturen bis zu etwa 200° zersetzt. Die Wolff-Kishner-Reduktion kann auch bei Raumtemperatur in Dimethylsulfoxid mit Hydrazin ausgeführt werden.

Man gelangt ferner zu Verbindungen der Formel I, worin R eine 4-$R^2$-1,2,3,6-tetrahydropyridylgruppe ist, indem man aus Verbindungen der Formel IV unter Ausbildung einer Doppelbindung HE abspaltet. Entsprechend der Definition von E kann es sich z.B. handeln um eine Abspaltung von Halogenwasserstoff, Wasser (Dehydratisierung), einer Carbonsäure oder einer anderen Säure, von Ammoniak oder von HCN. Die Ausgangsstoffe der Formel IV sind z.B. erhältlich durch Umsetzung von II ($X^1$ = X) mit einer Verbindung der Formel VII

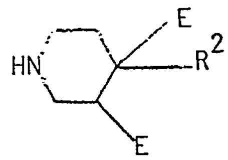

VII

worin E und $R^2$ die angegebenen Bedeutungen haben.

Falls einer der Reste E = Hal ist, kann dieser Substituent unter basischen Reaktionsbedingungen leicht eliminiert werden. Als Basen können verwendet werden: Alkalimetallhydroxide, Alkalimetallcarbonate, Alkoholate, wie z.B. Kalium-tert.-butylat, Amine, wie z.B. Dimethylanilin, Pyridin, Collidin oder Chinolin; als Lösungsmittel benutzt man z.B. Benzol, Toluol, Cyclohexan, THF oder tert.-Butanol. Die als Basen verwendeten Amine können auch im Überschuß als Lösungsmittel eingesetzt werden. Bedeutet der eine der Reste E eine OH-Gruppe, so benutzt man als wasserabspaltende Mittel vorzugsweise Säuren wie Essigsäure, Salzsäure oder Gemische beider. Der Zusatz eines Lösungsmittels (z.B. Wasser oder

Ethanol) kann von Vorteil sein. Die Eliminierung von Acyl-, Alkylsulfonyl- sowie Alkoxysulfonyl-oxy- oder Amino-Resten kann unter ähnlichen Bedingungen durchgeführt werden. Eine Eliminierung von Sulfonsäure-Resten, z.B. die der Mesylate oder Tosylate, erfolgt schonend durch Kochen in DMF oder Dimethylsulfoxid mit Alkalimetallcarbonaten, z.B. $Li_2CO_3$, oder mit Kaliumacetat. Ammoniak kann bereits durch Erhitzen der Salze der entsprechenden Aminoverbindungen (insbesondere der 4-Aminoderivate) abgespalten werden. In ähnlicher Weise kann HCN aus Verbindungen der Formel IV (eine Gruppe E = CN) durch Erhitzen abgespalten werden. Die Eliminierung von HE aus IV erfolgt allgemein bei Temperaturen zwischen 0 und etwa 250°, vorzugsweise zwischen 50 und 200°.

Weiterhin kann man gegebenenfalls eine Verbindung der Formel I nach an sich bekannten Methoden in eine andere Verbindung der Formel umwandeln.

So können Ether (O-Alkylderivate) gespalten werden, wobei die entsprechenden Hydroxyderivate entstehen. Z.B. kann man die Ether spalten durch Behandeln mit Dimethylsulfid-Bortribromid-Komplex, z.B. in Toluol, 1,2-Dichlorethan, THF oder Dimethylsulfoxid, durch Verschmelzen mit Pyridin- oder Anilinhydrohalogeniden, vorzugsweise Pyridinhydrochlorid, bei etwa 150-250°, mit HBr/Essigsäure oder mit Al-trihalogeniden in chlorierten Kohlenwasserstoffen wie 1,2-Dichlorethan.

Eine erhaltene Base der Formel I kann mit einer Säure in das zugehörige Säureadditionssalz übergeführt werden. Für diese Umsetzung eignen sich bevorzugt Säuren, die physiologisch unbedenkliche Salze liefern. So können anorganische Säuren verwendet werden, z.B. Schwefelsäure, Halogenwasserstoffsäuren wie Chlorwasserstoffsäure oder Bromwasserstoffsäure, Phosphorsäuren wie Orthophosphorsäuren, Salpetersäure, Sulfaminsäure, ferner organische Säuren, im einzelnen aliphatische, alicyclische, araliphatische, aromatische oder heterocyclische ein- oder mehrbasige Carbon-, Sulfon- oder Schwefelsäuren, wie Ameisensäure, Essigsäure, Propionsäure, Pivalinsäure, Diethylessigsäure, Malonsäure, Bernsteinsäure, Pimelinsäure, Fumarsäure, Maleinsäure, Milchsäure, Weinsäure, Äpfelsäure, Benzoesäure, Salicylsäure, 2-Phenylpropionsäure, Citronensäure, Gluconsäure, Ascorbinsäure, Nicotinsäure, Isonicotinsäure, Methan- oder Ethansulfonsäure, Ethandisulfonsäure, 2-Hydroxyethansulfonsäure, Benzolsulfonsäure, p-Toluolsulfonsäure, Naphthalin-mono- und -disulfonsäuren, Laurylschwefelsäure. Säureadditionssalze, die nicht physiologisch unbedenklich sind (z.B. Pikrate), können sich zur Isolierung und Aufreinigung von Basen der Formel I eignen.

Die freien Basen der Formel I können, falls gewünscht, aus ihren Salzen durch Behandlung mit starken Basen wie Natrium- oder Kaliumhydroxid, Natrium- oder Kaliumcarbonat in Freiheit gesetzt werden.

Gegenstand der Erfindung ist ferner die Verwendung der Verbindungen der Formel I und ihrer physiologisch unbedenklichen Salze zur Herstellung pharmazeutischer Zubereitungen, insbesondere auf nicht-chemischem Wege. Hierbei können sie zusammen mit mindestens einem Träger- oder Hilfsstoff und gegebenenfalls in Kombination mit einem oder mehreren weiteren Wirkstoff(en) in eine geeignete Dosierungsform gebracht werden.

Gegenstand der Erfindung sind ferner Mittel, insbesondere pharmazeutische Zubereitungen, enthaltend mindestens eine Verbindung der Formel I und/oder eines ihrer physiologisch unbedenklichen Salze. Diese Zubereitungen können als Arzneimittel in der Human- oder Veterinärmedizin eingesetzt werden. Als Trägersubstanzen kommen organische oder anorganische Stoffe in Frage, die sich für die enterale (z.B. orale), parenterale oder topische Applikation eignen und mit den neuen Verbindungen nicht reagieren, beispielsweise Wasser, pflanzliche Öle, Benzylalkohole, Polyethylenglykole, Gelatine, Kohlehydrate wie Lactose oder Stärke, Magnesiumstearat, Talk, Vaseline. Zur enteralen Applikation dienen insbesondere Tabletten, Dragees, Kapseln, Sirupe, Säfte, Tropfen oder Suppositorien, zur parenteralen Applikation Lösungen, vorzugsweise ölige oder wäßrige Lösungen, ferner Suspensionen, Emulsionen oder Implantate, für die topische Anwendung Salben, Cremes oder Puder. Die neuen Verbindungen können auch lyophilisiert und die erhaltenen Lyophilisate z.B. zur Herstellung von Injektionspräparaten verwendet werden.

Die angegebenen Zubereitungen können sterilisiert sein und/oder Hilfsstoffe wie Gleit-, Konservierungs-, Stabilisierungs- und/oder Netzmittel, Emulgatoren, Salze zur Beeinflussung des osmotischen Druckes, Puffersubstanzen, Farb-, Geschmacks- und/oder Aromastoffe enthalten. Sie können, falls erwünscht, auch einen oder mehrere weitere Wirkstoffe enthalten, z.B. ein oder mehrere Vitamine.

Verbindungen der Formel I und ihre physiologisch unbedenklichen Salze können verwendet werden bei der therapeutischen Behandlung des menschlichen oder tierischen Körpers und bei der Bekämpfung von Krankheiten, insbesondere von Schizophrenie und psychoreaktiven Störungen und Psychopathien, von Depressionen, von schweren chronischen Schmerzen und von Krankheiten, die mit erhöhtem Blutdruck einhergehen.

Weiterhin können die Verbindungen bei der Behandlung extrapyramidaler Störungen Anwendung finden.

Dabei werden die erfindungsgemäßen Substanzen in der Regel in Analogie zu bekannten, im Handel befindlichen Präparaten (Thioridazin, Haloperidol) verabreicht, vorzugsweise in Dosierungen zwischen etwa 0,2 und 500 mg, insbesondere zwischen 0,2 und 50 mg pro Dosierungseinheit. Die tägliche Dosierung liegt vorzugsweise zwischen etwa 0,003 und 10 mg/kg Körpergewicht.

Die spezielle Dosis für jeden bestimmten Patienten hängt jedoch von den verschiedensten Faktoren ab, beispielsweise von der Wirksamkeit der eingesetzten speziellen Verbindung, vom Alter, Körpergewicht, allgemeinen Gesundheitszustand, Geschlecht, von der Kost, vom Verabfolgungszeitpunkt und -

weg, von der Ausscheidungsgeschwindigkeit, Arzneistoffkombination und Schwere der jeweiligen Erkrankung, welcher die Therapie gilt. Die orale Applikation ist bevorzugt.

In den nachstehenden Beispielen bedeutet "übliche Aufarbeitung": Man gibt, falls erforderlich, Wasser hinzu, extrahiert mit Dichlormethan, trennt ab, trocknet die organische Phase über Natriumsulfat, filtriert, dampft ein und reinigt durch Chromatographie an Kieselgel und/oder durch Kristallisation. Temperaturen sind in °C angegeben.

Beispiel 1

Man kocht eine Lösung von 24 g 2-Chlormethyl-4-phenylpyridinhydrochlorid [F. 174-176°; erhältlich durch Reduktion von 4-Phenylpyridin-2-carbonsäure mit $LiAlH_4$ zu 2-Hydroxymethyl-4-phenyl-pyridin (F. 183-185°) und Reaktion mit $SOCl_2$], 16 g 4-Phenyl-1,2,3,6-tetrahydropyridin und 22 ml Triethylamin in 300 ml Acetonitril 12 Stunden, arbeitet wie üblich auf und erhält 2-(4-Phenyl-1,2,3,6-tetrahydropyridyl-methyl)-4-phenyl-pyridin ("P"), Dihydrochlorid, F. 223° (Zers.).

Analog erhält man aus den entsprechenden Methansulfonaten, Chloriden oder Bromiden:

2-(4-Phenyl-1,2,3,6-tetrahydropyridyl-methyl)-4-o-tolylpyridin
2-(4-Phenyl-1,2,3,6-tetrahydropyridyl-methyl)-4-m-tolylpyridin
2-(4-Phenyl-1,2,3,6-tetrahydropyridyl-methyl)-4-p-tolylpyridin
2-(4-Phenyl-1,2,3,6-tetrahydropyridyl-methyl)-4-m-methoxyphenyl-pyridin
2-(4-Phenyl-1,2,3,6-tetrahydropyridyl-methyl)-4-p-methoxyphenyl-pyridin
2-(4-Phenyl-1,2,3,6-tetrahydropyridyl-methyl)-4-p-fluorphenyl-pyridin
2-(4-Phenyl-1,2,3,6-tetrahydropyridyl-methyl)-4-p-chlorphenyl-pyridin
2-(4-Phenyl-1,2,3,6-tetrahydropyridyl-methyl)-4-m-hydroxyphenyl-pyridin
2-(4-Phenyl-1,2,3,6-tetrahydropyridyl-methyl)-4-p-hydroxyphenyl-pyridin
2-(4-Phenyl-1,2,3,6-tetrahydropyridyl-methyl)-4-m-trifluormethylphenyl-pyridin
2-(4-Phenyl-1,2,3,6-tetrahydropyridyl-methyl)-4-(3,4-dimethoxyphenyl)-pyridin
2-(4-Phenyl-1,2,3,6-tetrahydropyridyl-methyl)-4-(2-thienyl)-pyridin
2-(4-o-Tolyl-1,2,3,6-tetrahydropyridyl-methyl)-4-phenyl-pyridin
2-(4-m-Tolyl-1,2,3,6-tetrahydropyridyl-methyl)-4-phenyl-pyridin
2-(4-p-Tolyl-1,2,3,6-tetrahydropyridyl-methyl)-4-phenyl-pyridin
2-(4-p-Methoxyphenyl-1,2,3,6-tetrahydropyridyl-methyl)-4-phenyl-pyridin
2-(4-p-Fluorphenyl-1,2,3,6-tetrahydropyridyl-methyl)-4-phenyl-pyridin
2-(4-p-Chlorphenyl-1,2,3,6-tetrahydropyridyl-methyl)-4-phenyl-pyridin
2-(4-m-Trifluormethylphenyl-1,2,3,6-tetrahydropyridyl-methyl)-4-phenyl-pyridin
2-[4-(4-Chlor-3-trifluormethylphenyl-1,2,3,6-tetrahydropyridyl-methyl]-4-phenyl-pyridin
2-[4-(2-Thienyl)-1,2,3,6-tetrahydropyridyl-methyl]-4-phenyl-pyridin
2-[4-(2-Thienyl)-1,2,3,6-tetrahydropyridyl-methyl]-4-m-tolyl-pyridin
2-[4-(2-Thienyl)-1,2,3,6-tetrahydropyridyl-methyl]-4-p-tolyl-pyridin
2-[4-(2-Thienyl)-1,2,3,6-tetrahydropyridyl-methyl]-4-p-fluorphenyl-pyridin
2-[4-(3-Thienyl)-1,2,3,6-tetrahydropyridyl-methyl]-4-phenyl-pyridin
2-[1-(4-Phenyl-1,2,3,6-tetrahydropyridyl)-ethyl]-4-phenyl-pyridin
2-[1-(4-Phenyl-1,2,3,6-tetrahydropyridyl)-ethyl]-4-m-methoxyphenyl-pyridin
2-[1-(4-Phenyl-1,2,3,6-tetrahydropyridyl)-ethyl]-4-p-methoxyphenyl-pyridin
2-[2-(4-Phenyl-1,2,3,6-tetrahydropyridyl)-ethyl]-4-phenyl-pyridin
2-[2-(4-Phenyl-1,2,3,6-tetrahydropyridyl)-ethyl]-4-m-methoxyphenyl-pyridin
2-[2-(4-Phenyl-1,2,3,6-tetrahydropyridyl)-ethyl]-4-p-methoxyphenyl-pyridin
2-[3-(4-Phenyl-1,2,3,6-tetrahydropyridyl)-propyl]-4-phenyl-pyridin
2-[3-(4-Phenyl-1,2,3,6-tetrahydropyridyl)-propyl]-4-m-methoxyphenyl-pyridin
2-[3-(4-Phenyl-1,2,3,6-tetrahydropyridyl)-propyl]-4-p-methoxyphenyl-pyridin
2-(4-p-Fluorphenyl-1,2,3,6-tetrahydropyridyl-methyl)-4-p-fluorphenyl-pyridin
2-[1-(4-p-Fluorphenyl-1,2,3,6-tetrahydropyridyl)-ethyl]-4-p-fluorphenyl-pyridin
2-[2-(4-p-Fluorphenyl-1,2,3,6-tetrahydropyridyl)-ethyl]-4-p-fluorphenyl-pyridin
2-[3-(4-p-Fluorphenyl-1,2,3,6-tetrahydropyridyl)-propyl]-4-p-fluorphenyl-pyridin
2-[2-(4-(2-Thienyl)-1,2,3,6-tetrahydropyridyl)-ethyl]-4-phenyl-pyridin
2-[3-(4-(2-Thienyl)-1,2,3,6-tetrahydropyridyl)-propyl]-4-phenyl-pyridin

2-Phenyl-6-(4-phenylpiperidinomethyl)-pyridin
2-Phenyl-6-(4-p-fluorphenylpiperidinomethyl)-pyridin
2-Phenyl-6-(4-p-chlorphenylpiperidinomethyl)-pyridin

2-p-Fluorphenyl-6-(4-phenylpiperidinomethyl)-pyridin
2-p-Fluorphenyl-6-(4-p-fluorphenylpiperidinomethyl)-pyridin
2-p-Fluorphenyl-6-(4-p-chlorphenylpiperidinomethyl)-pyridin

2-p-Chlorphenyl-6-(4-phenylpiperidinomethyl)-pyridin
2-p-Chlorphenyl-6-(4-p-fluorphenylpiperidinomethyl)-pyridin
2-p-Chlorphenyl-6-(4-p-chlorphenylpiperidinomethyl)-pyridin

2-Phenyl-4-(4-phenylpiperidinomethyl)-pyridin,
Dihydrochlorid, F. 241-243°
2-Phenyl-4-(4-p-fluorphenylpiperidinomethyl)-pyridin
2-Phenyl-4-(4-p-chlorphenylpiperidinomethyl)-pyridin

2-p-Fluorphenyl-4-(4-phenylpiperidinomethyl)-pyridin
2-p-Fluorphenyl-4-(4-p-fluorphenylpiperidinomethyl)-pyridin
2-p-Fluorphenyl-4-(4-p-chlorphenylpiperidinomethyl)-pyridin

2-p-Chlorphenyl-4-(4-phenylpiperidinomethyl)-pyridin
2-p-Chlorphenyl-4-(4-p-fluorphenylpiperidinomethyl)-pyridin
2-p-Chlorphenyl-4-(4-p-chlorphenylpiperidinomethyl)-pyridin

3-Phenyl-5-(4-phenylpiperidinomethyl)-pyridin, Dihydrochlorid-trihydrat, F. 99-101°; Malonat; F.167° (Zers.);
Maleat, F. 194°; Difumarat, F. 181 ; Succinat, F. 121° 3-Phenyl-5-(4-p-fluorphenylpiperidinomethyl)-pyridin,
Dihydrochlorid, F. 230-233°
3-Phenyl-5-(4-p-chlorphenylpiperidinomethyl)-pyridin

3-p-Fluorphenyl-5-(4-phenylpiperidinomethyl)-pyridin,
Malonat, F. 142-143°
3-p-Fluorphenyl-5-(4-p-fluorphenylpiperidinomethyl)-pyridin
3-p-Fluorphenyl-5-(4-p-chlorphenylpiperidinomethyl)-pyridin

3-p-Chlorphenyl-5-(4-phenylpiperidinomethyl)-pyridin
3-p-Chlorphenyl-5-(4-p-fluorphenylpiperidinomethyl)-pyridin
3-p-Chlorphenyl-5-(4-p-chlorphenylpiperidinomethyl)-pyridin

2-(4-Phenylpiperidinomethyl)-4-phenyl-pyridin,
Dihydrochlorid-dihydrat. F. 214°
2-(4-p-Fluorphenylpiperidinomethyl)-4-phenyl-pyridin
2-(4-p-Chlorphenylpiperidinomethyl)-4-phenyl-pyridin

2-(4-Phenylpiperidinomethyl)-4-p-fluorphenyl-pyridin
2-(4-p-Fluorphenylpiperidinomethyl)-4-p-fluorphenyl-pyridin
2-(4-p-Chlorphenylpiperidinomethyl)-4-p-fluorphenyl-pyridin

2-(4-Phenylpiperidinomethyl)-4-p-chlorphenyl-pyridin
2-(4-p-Fluorphenylpiperidinomethyl)-4-p-chlorphenylpyridin
2-(4-p-Chlorphenylpiperidinomethyl)-4-p-chlorphenyl-pyridin

3-Phenyl-5-[2-(4-phenylpiperidino)-ethyl]-pyridin,F.89-91°
3-Phenyl-5-[2-(4-p-fluorphenylpiperidino)-ethyl]-pyridin
3-Phenyl-5-[2-(4-p-chlorphenylpiperidino)-ethyl]-pyridin

3-p-Fluorphenyl-5-[2-(4-phenylpiperidino)-ethyl]-pyridin
3-p-Fluorphenyl-5-[2-(4-p-fluorphenylpiperidino)-ethyl]-pyridin
3-p-Fluorphenyl-5-[2-(4-p-chlorphenylpiperidino)-ethyl]-pyridin

3-p-Chlorphenyl-5-[2-(4-phenylpiperidino)-ethyl]-pyridin
3-p-Chlorphenyl-5-[2-(4-p-fluorphenylpiperidino)-ethyl]-pyridin
3-p-Chlorphenyl-5-[2-(4-p-chlorphenylpiperidino)-ethyl]-pyridin

2-Phenyl-6-(4-phenyl-1,2,3,6-tetrahydropyridyl-methyl)-pyridin, Hydrochlorid, F. 206-208°
2-Phenyl-6-(4-p-fluorphenyl-1,2,3,6-tetrahydropyridyl-methyl)-pyridin
2-Phenyl-6-(4-p-chlorphenyl-1,2,3,6-tetrahydropyridyl-methyl)-pyridin

2-p-Fluorphenyl-6-(4-phenyl-1,2,3,6-tetrahydropyridyl-methyl)-pyridin
2-p-Fluorphenyl-6-(4-p-fluorphenyl-1,2,3,6-tetrahydropyridyl-methyl)-pyridin
2-p-Fluorphenyl-6-(4-p-chlorphenyl-1,2,3,6-tetrahydropyridyl-methyl)-pyridin

2-p-Chlorphenyl-6-(4-phenyl-1,2,3,6-tetrahydropyridyl-methyl)-pyridin
2-p-Chlorphenyl-6-(4-p-fluorphenyl-1,2,3,6-tetrahydropyridyl-methyl)-pyridin
2-p-Chlorphenyl-6-(4-p-chlorphenyl-1,2,3,6-tetrahydropyridyl-methyl)-pyridin

2-Phenyl-4-(4-phenyl-1,2,3,6-tetrahydropyridyl-methyl)-pyridin
2-Phenyl-4-(4-p-fluorphenyl-1,2,3,6-tetrahydropyridyl-methyl)-pyridin
2-Phenyl-4-(4-p-chlorphenyl-1,2,3,6-tetrahydropyridyl-methyl)-pyridin

2-p-Fluorphenyl-4-(4-phenyl-1,2,3,6-tetrahydropyridyl-methyl)-pyridin
2-p-Fluorphenyl-4-(4-p-fluorphenyl-1,2,3,6-tetrahydropyridyl-methyl)-pyridin
2-p-Fluorphenyl-4-(4-p-chlorphenyl-1,2,3,6-tetrahydropyridyl-methyl)-pyridin

2-p-Chlorphenyl-4-(4-phenyl-1,2,3,6-tetrahydropyridyl-methyl)-pyridin
2-p-Chlorphenyl-4-(4-p-fluorphenyl-1,2,3,6-tetrahydropyridyl-methyl)-pyridin
2-p-Chlorphenyl-4-(4-p-chlorphenyl-1,2,3,6-tetrahydropyridyl-methyl)-pyridin

2-Phenyl-6-(4-Phenyl-4-hydroxypiperidinomethyl)-pyridin
2-Phenyl-6-(4-p-Fluorphenyl-4-hydroxypiperidinomethyl)-pyridin
2-Phenyl-6-(4-p-Chlorphenyl-4-hydroxypiperidinomethyl)-pyridin

2-p-Fluorphenyl-6-(4-phenyl-4-hydroxypiperidinomethyl)-pyridin
2-p-Fluorphenyl-6-(4-p-fluorphenyl-4-hydroxypiperidinomethyl)-pyridin
2-p-Fluorphenyl-6-(4-p-chlorphenyl-4-hydroxypiperidinomethyl)-pyridin

2-p-Chlorphenyl-6-(4-phenyl-4-hydroxypiperidinomethyl)-pyridin
2-p-Chlorphenyl-6-(4-p-fluorphenyl-4-hydroxypiperidinomethyl)-pyridin
2-p-Chlorphenyl-6-(4-p-chlorphenyl-4-hydroxypiperidinomethyl)-pyridin

2-Phenyl-4-(4-phenyl-4-hydroxypiperidinomethyl)-pyridin
2-Phenyl-4-(4-p-fluorphenyl-4-hydroxypiperidinomethyl)-pyridin
2-Phenyl-4-(4-p-chlorphenyl-4-hydroxypiperidinomethyl)-pyridin, F. 149-151°

2-p-Fluorphenyl-4-(4-phenyl-4-hydroxypiperidinomethyl)-pyridin
2-p-Fluorphenyl-4-(4-p-fluorphenyl-4-hydroxypiperidinomethyl)-pyridin
2-p-Fluorphenyl-4-(4-p-chlorphenyl-4-hydroxypiperidinomethyl)-pyridin

2-p-Chlorphenyl-4-(4-phenyl-4-hydroxypiperidinomethyl)-pyridin
2-p-Chlorphenyl-4-(4-p-fluorphenyl-4-hydroxypiperidinomethyl)-pyridin
2-p-Chlorphenyl-4-(4-p-chlorphenyl-4-hydroxypiperidinomethyl)-pyridin

3-Phenyl-5-(4-phenyl-4-hydroxypiperidinomethyl)-pyridin
3-Phenyl-5-(4-p-fluorphenyl-4-hydroxypiperidinomethyl)-pyridin
3-Phenyl-5-(4-p-chlorphenyl-4-hydroxypiperidinomethyl)-pyridin, Hydrochlorid, F. 246-247°

3-p-Fluorphenyl-5-(4-phenyl-4-hydroxypiperidinomethyl)-pyridin, F. 157-159°
3-p-Fluorphenyl-5-(4-p-fluorphenyl-4-hydroxypiperidinomethyl)-pyridin
3-p-Fluorphenyl-5-(4-p-chlorphenyl-4-hydroxypiperidinomethyl)-pyridin, Hydrochlorid, F. 233-234°

3-p-Chlorphenyl-5-(4-phenyl-4-hydroxypiperidinomethyl)-pyridin
3-p-Chlorphenyl-5-(4-p-fluorphenyl-4-hydroxypiperidinomethyl)-pyridin
3-p-Chlorphenyl-5-(4-p-chlorphenyl-4-hydroxypiperidinomethyl)-pyridin

2-(4-Phenyl-4-hydroxypiperidinomethyl)-4-phenyl-pyridin
2-(4-p-Fluorphenyl-4-hydroxypiperidinomethyl)-4-phenyl-pyridin
2-(4-p-Chlorphenyl-4-hydroxypiperidinomethyl)-4-phenyl-pyridin

2-(4-Phenyl-4-hydroxypiperidinomethyl)-4-p-fluorphenyl-pyridin
2-(4-p-Fluorphenyl-4-hydroxypiperidinomethyl)-4-p-fluorphenyl-pyridin
2-(4-p-Chlorphenyl-4-hydroxypiperidinomethyl)-4-p-fluorphenyl-pyridin

2-(4-Phenyl-4-hydroxypiperidinomethyl)-4-p-chlorphenyl-pyridin
2-(4-p-Fluorphenyl-4-hydroxypiperidinomethyl)-4-p-chlorphenyl-pyridin
2-(4-p-Chlorphenyl-4-hydroxypiperidinomethyl)-4-p-chlorphenyl-pyridin

2-Phenyl-6-(4-benzoylpiperidinomethyl)-pyridin

2-Phenyl-6-(4-p-fluorbenzoylpiperidinomethyl)-pyridin
2-Phenyl-6-(4-p-chlorbenzoylpiperidinomethyl)-pyridin

2-p-Fluorphenyl-6-(4-benzoylpiperidinomethyl)-pyridin
2-p-Fluorphenyl-6-(4-p-fluorbenzoylpiperidinomethyl)-pyridin
2-p-Fluorphenyl-6-(4-p-chlorbenzoylpiperidinomethyl)-pyridin

2-p-Chlorphenyl-6-(4-benzoylpiperidinomethyl)-pyridin
2-p-Chlorphenyl-6-(4-p-fluorbenzoylpiperidinomethyl)-pyridin
2-p-Chlorphenyl-6-(4-p-chlorbenzoylpiperidinomethyl)-pyridin

2-Phenyl-4-(4-benzoylpiperidinomethyl)-pyridin
2-Phenyl-4-(4-p-fluorbenzoylpiperidinomethyl)-pyridin
2-Phenyl-4-(4-p-chlorbenzoylpiperidinomethyl)-pyridin

2-p-Fluorphenyl-4-(4-benzoylpiperidinomethyl)-pyridin
2-p-Fluorphenyl-4-(4-p-fluorbenzoylpiperidinomethyl)-pyridin
2-p-Fluorphenyl-4-(4-p-chlorbenzoylpiperidinomethyl)-pyridin

2-p-Chlorphenyl-4-(4-benzoylpiperidinomethyl)-pyridin
2-p-Chlorphenyl-4-(4-p-fluorbenzoylpiperidinomethyl)-pyridin
2-p-Chlorphenyl-4-(4-p-chlorbenzoylpiperidinomethyl)-pyridin

3-Phenyl-5-(4-benzoylpiperidinomethyl)-pyridin
3-Phenyl-5-(4-p-fluorbenzoylpiperidinomethyl)-pyridin,F.91-93°
3-Phenyl-5-(4-p-chlorbenzoylpiperidinomethyl)-pyridin

3-p-Fluorphenyl-5-(4-benzoylpiperidinomethyl)-pyridin
3-p-Fluorphenyl-5-(4-p-fluorbenzoylpiperidinomethyl)-pyridin
3-p-Fluorphenyl-5-(4-p-chlorbenzoylpiperidinomethyl)-pyridin

3-p-Chlorphenyl-5-(4-benzoylpiperidinomethyl)-pyridin
3-p-Chlorphenyl-5-(4-p-fluorbenzoylpiperidinomethyl)-pyridin
3-p-Chlorphenyl-5-(4-p-chlorbenzoylpiperidinomethyl)-pyridin

2-(4-Benzoylpiperidinomethyl)-4-phenyl-pyridin
2-(4-p-Fluorbenzoylpiperidinomethyl)-4-phenyl-pyridin
2-(4-p-Chlorbenzoylpiperidinomethyl)-4-phenyl-pyridin

2-(4-Benzoylpiperidinomethyl)-4-p-fluorphenyl-pyridin
2-(4-p-Fluorbenzoylpiperidinomethyl)-4-p-fluorphenyl-pyridin
2-(4-p-Chlorbenzoylpiperidinomethyl)-4-p-fluorphenyl-pyridin

2-(4-Benzoylpiperidinomethyl)-4-p-chlorphenyl-pyridin
2-(4-p-Fluorbenzoylpiperidinomethyl)-4-p-chlorphenyl-pyridin
2-(4-p-Chlorbenzoylpiperidinomethyl)-4-p-chlorphenyl-pyridin

2-Phenyl-6-[4-(2-oxobenzimidazolin-1-yl)-piperidinomethyl]-pyridin
2-Phenyl-6-[4-(2-oxo-5-fluorbenzimidazolin-1-yl)-piperidinomethyl]-pyridin
2-Phenyl-6-[4-(2-oxo-5-chlorbenzimidazolin-1-yl)-piperidinomethyl]-pyridin

2-p-Fluorphenyl-6-[4-(2-oxobenzimidazolin-1-yl)-piperidinomethyl]-pyridin
2-p-Fluorphenyl-6-[4-(2-oxo-5-fluorbenzimidazolin-1-yl)-piperidinomethyl]-pyridin
2-p-Fluorphenyl-6-[4-(2-oxo-5-chlorbenzimidazolin-1-yl)-piperidinomethyl]-pyridin

2-p-Chlorphenyl-6-[4-(2-oxobenzimidazolin-1-yl)-piperidinomethyl]-pyridin
2-p-Chlorphenyl-6-[4-(2-oxo-5-fluorbenzimidazolin-1-yl)-piperidinomethyl]-pyridin
2-p-Chlorphenyl-6-[4-(2-oxo-5-chlorbenzimidazolin-1-yl)-piperidinomethyl]-pyridin

2-Phenyl-4-[4-(2-oxobenzimidazolin-1-yl)-piperidinomethyl]-pyridin, F. 203-205°
2-Phenyl-4-[4-(2-oxo-5-fluorbenzimidazolin-1-yl)-piperidinomethyl]-pyridin
2-Phenyl-4-[4-(2-oxo-5-chlorbenzimidazolin-1-yl)-piperidinomethyl]-pyridin

2-p-Fluorphenyl-4-[4-(2-oxobenzimidazolin-1-yl)-piperidinomethyl]-pyridin
2-p-Fluorphenyl-4-[4-(2-oxo-5-fluorbenzimidazolin-1-yl)-piperidinomethyl]-pyridin

2-p-Fluorphenyl-4-[4-(2-oxo-5-chlorbenzimidazolin-1-yl)-piperidinomethyl]-pyridin

2-p-Chlorphenyl-4-[4-(2-oxobenzimidazolin-1-yl)-piperidinomethyl]-pyridin
2-p-Chlorphenyl-4-[4-(2-oxo-5-fluorbenzimidazolin-1-yl)-piperidinomethyl]-pyridin
2-p-Chlorphenyl-4-[4-(2-oxo-5-chlorbenzimidazolin-1-yl)-piperidinomethyl]-pyridin

3-Phenyl-5-[4-(2-oxobenzimidazolin-1-yl)-piperidinomethyl]-pyridin, F. 186-188°
3-Phenyl-5-[4-(2-oxo-5-fluorbenzimidazolin-1-yl)-piperidinomethyl]-pyridin
3-Phenyl-5-[4-(2-oxo-5-chlorbenzimidazolin-1-yl)-piperidinomethyl]-pyridin

3-p-Fluorphenyl-5-[4-(2-oxobenzimidazolin-1-yl)-piperidinomethyl]-pyridin, Dihydrochlorid, F. 229-231°
3-p-Fluorphenyl-5-[4-(2-oxo-5-fluorbenzimidazolin-1-yl)-piperidnomethyl]-pyridin
3-p-Fluorphenyl-5-[4-(2-oxo-5-chlorbenzimidazolin-1-yl)-piperidinomethyl]-pyridin

3-p-Chlorphenyl-5-[4-(2-oxobenzimidazolin-1-yl)-piperidinomethyl]-pyridin
3-p-Chlorphenyl-5-[4-(2-oxo-5-fluorbenzimidazolin-1-yl)-piperidinomethyl]-pyridin
3-p-Chlorphenyl-5-[4-(2-oxo-5-chlorbenzimidazolin-1-yl)-piperidinomethyl]-pyridin

2-[4-(2-Oxobenzimidazolin-1-yl)-piperidinomethyl]-4-phenyl-pyridin
2-[4-(2-Oxo-5-fluorbenzimidazolin-1-yl)-piperidinomethyl]-4-phenyl-pyridin
2-[4-(2-Oxo-5-chlorbenzimidazolin-1-yl)-piperidinomethyl)-4-phenyl-pyridin

2-[4-(2-Oxobenzimidazolin-1-yl)-piperidinomethyl]-4-p-fluorphenyl-pyridin
2-[4-(2-Oxo-5-fluorbenzimidazolin-1-yl)-piperidinomethyl]-4-p-fluorphenyl-pyridin
2-[4-(2-Oxo-5-chlorbenzimidazolin-1-yl)-piperidinomethyl]-4-p-fluorphenyl-pyridin

2-[4-(2-Oxobenzimidazolin-1-yl)-piperidinomethyl]-4-p-chlorphenyl-pyridin
2-[4-(2-Oxo-5-fluorbenzimidazolin-1-yl)-piperidinomethyl]-4-p-chlorphenyl-pyridin
2-[4-(2-Oxo-5-chlorbenzimidazolin-1-yl)-piperidinomethyl]-4-p-chlorphenyl-pyridin

8-(2-Phenyl-6-pyridylmethyl)-1-phenyl-4-oxo-1,3,8-triazaspiro[4,5]decan
8-(2-Phenyl-6-pyridylmethyl)-1-p-fluorphenyl-4-oxo-1,3,8-triazaspiro[4,5]decan
8-(2-Phenyl-6-pyridylmethyl)-1-p-chlorphenyl-4-oxo-1,3,8-triazaspiro[4,5]decan

8-(2-p-Fluorphenyl-6-pyridylmethyl)-1-phenyl-4-oxo-1,3,8-triazaspiro[4,5]decan
8-(2-p-Fluorphenyl-6-pyridylmethyl)-1-p-fluorphenyl-4-oxo-1,3,8-triazaspiro[4,5]decan
8-(2-p-Fluorphenyl-6-pyridylmethyl)-1-p-chlorphenyl-4-oxo-1,3,8-triazaspiro[4,5]decan

8-(2-p-Chlorphenyl-6-pyridylmethyl)-1-phenyl-4-oxo-1,3,8-triazaspiro[4,5]decan
8-(2-p-Chlorphenyl-6-pyridylmethyl)-1-p-fluorphenyl-4-oxo-1,3,8-triazaspiro[4,5]decan
8-(2-p-Chlorphenyl-6-pyridylmethyl)-1-p-chlorphenyl-4-oxo-1,3,8-triazaspiro[4,5]decan

8-(2-Phenyl-4-pyridylmethyl)-1-phenyl-4-oxo-1,3,8-triazaspiro[4,5]decan
8-(2-Phenyl-4-pyridylmethyl)-1-p-fluorphenyl-4-oxo-1,3,8-triazaspiro[4,5]decan
8-(2-Phenyl-4-pyridylmethyl)-1-p-chlorphenyl-4-oxo-1,3,8-triazaspiro[4,5]decan

8-(2-p-Fluorphenyl-4-pyridylmethyl)-1-phenyl-4-oxo-1,3,8-triazaspiro[4,5]decan
8-(2-p-Fluorphenyl-4-pyridylmethyl)-1-p-fluorphenyl-4-oxo-1,3,8-triazaspiro[4,5]decan
8-(2-p-Fluorphenyl-4-pyridylmethyl)-1-p-chlorphenyl-4-oxo-1,3,8-triazaspiro[4,5]decan

8-(2-p-Chlorphenyl-4-pyridylmethyl)-1-phenyl-4-oxo-1,3,8-triazaspiro[4,5]decan
8-(2-p-Chlorphenyl-4-pyridylmethyl)-1-p-fluorphenyl-4-oxo-1,3,8-triazaspiro[4,5]decan
8-(2-p-Chlorphenyl-4-pyridylmethyl)-1-p-chlorphenyl-4-oxo-1,3,8-triazaspiro[4,5]decan

8-(3-Phenyl-5-pyridylmethyl)-1-phenyl-4-oxo-1,3,8-triazaspiro[4,5]decan, F. 223-225°
8-(3-Phenyl-5-pyridylmethyl)-1-p-fluorphenyl-4-oxo-1,3,8-triazaspiro[4,5]decan
8-(3-Phenyl-5-pyridylmethyl)-1-p-chlorphenyl-4-oxo-1,3,8-triazaspiro[4,5]decan

8-(3-p-Fluorphenyl-5-pyridylmethyl)-1-phenyl-4-oxo-1,3,8-triazaspiro[4,5]decan
8-(3-p-Fluorphenyl-5-pyridylmethyl)-1-p-fluorphenyl-4-oxo-1,3,8-triazaspiro[4,5]decan
8-(3-p-Fluorphenyl-5-pyridylmethyl)-1-p-chlorphenyl-4-oxo-1,3,8-triazaspiro[4,5]decan

8-(3-p-Chlorphenyl-5-pyridylmethyl)-1-phenyl-4-oxo-1,3,8-triazaspiro[4,5]decan
8-(3-p-Chlorphenyl-5-pyridylmethyl)-1-p-fluorphenyl-4-oxo-1,3,8-triazaspiro[4,5]decan
8-(3-p-Chlorphenyl-5-pyridylmethyl)-1-p-chlorphenyl-4-oxo-1,3,8-triazaspiro[4,5]decan

8-(4-Phenyl-2-pyridylmethyl)-1-phenyl-4-oxo-1,3,8-triazaspiro[4,5]decan
8-(4-Phenyl-2-pyridylmethyl)-1-p-fluorphenyl-4-oxo-1,3,8-triazaspiro[4,5]decan
8-(4-Phenyl-2-pyridylmethyl)-1-p-chlorphenyl-4-oxo-1,3,8-triazaspiro[4,5]decan

8-(4-p-Fluorphenyl-2-pyridylmethyl)-1-phenyl-4-oxo-1,3,8-triazaspiro[4,5]decan
8-(4-p-Fluorphenyl-2-pyridylmethyl)-1-p-fluorphenyl-4-oxo-1,3,8-triazaspiro[4,5]decan
8-(4-p-Fluorphenyl-2-pyridylmethyl)-1-p-chlorphenyl-4-oxo-1,3,8-triazaspiro[4,5]decan

8-(4-p-Chlorphenyl-2-pyridylmethyl)-1-phenyl-4-oxo-1,3,8-triazaspiro[4,5]decan
8-(4-p-Chlorphenyl-2-pyridylmethyl)-1-p-fluorphenyl-4-oxo-1,3,8-triazaspiro[4,5]decan
8-(4-p-Chlorphenyl-2-pyridylmethyl)-1-p-chlorphenyl-4-oxo-1,3,8-triazaspiro[4,5]decan.

Beispiel 2

Ein Gemisch von 1,84 g 2-Aminomethyl-4-phenylpyridin (erhältlich durch $LiAlH_4$-Reduktion von 2-Cyan-4-phenyl-pyridin) und 2,15 g 1,5-Dichlor-3-phenyl-2-penten in 40 ml Aceton und 40 ml Wasser wird 24 Stunden gekocht und wie üblich aufgearbeitet. Man erhält "P", Dihydrochlorid, F. 223° (Zers.).

Beispiel 3

Eine Suspension von 4-Phenyl-1-(4-phenylpicolinoyl)-1,2,3,6-tetrahydropyridin (erhältlich durch Reaktion von 4-Phenyl-picolinsäure mit 4-Phenyl-1,2,3,6-tetrahydropyridin in Gegenwart von Carbonyldiimidazol in THF) in 200 ml THF wird zu einer Suspension von 3,8 g $LiAlH_4$ in 200 ml THF unter Rühren hinzugetropft. Man rührt noch 2 Stunden bei 20°, arbeitet wie üblich auf und erhält "P", Dihydrochlorid, F. 223° (Zers.).

Analog erhält man aus den entsprechenden Säureamiden die übrigen in Beispiel 1 genannten Verbindungen.

Beispiel 4

Man erhitzt 3,44 g 3-(4-Hydroxy-4-phenyl-1-piperidyl-methyl)-4-phenyl-pyridin mit 40 ml 1 n Salzsäure 2 Stunden auf 50°, arbeitet wie üblich auf und erhält "P", Dihydrochlorid, F. 223° (Zers.).

Beispiel 5

Ein Gemisch von 10 g 2-(4-Phenyl-1,2,3,6-tetrahydropyridyl-methyl)-4-m-methoxyphenyl-pyridin und 10 g Pyridinhydrochlorid wird 3 Stunden bei 160° gerührt. Nach üblicher Aufarbeitung erhält man 2-(4-Phenyl-1,2,3,6-tetrahydropyridyl-methyl)-4-m-hydroxyphenyl-pyridin.

Beispiel 6

Man tropft eine Suspension von 3,56 g 2-(4-Phenyl-1,2,3,6-tetrahydropyridyl-methyl)-6-m-methoxyphenyl-pyridin in 50 ml 1,2-Dichlorethan zu einer siedenden Lösung von 15,6 g Dimethylsulfid-Bortribromid-Komplex in 50 ml 1,2-Dichlorethan, kocht noch 30 Minuten, arbeitet wie üblich auf und erhält 2-(4-Phenyl-1,2,3,6-tetrahydropyridyl-methyl)-6-m-hydroxyphenyl-pyridin.

Die nachstehenden Beispiele betreffen pharmazeutische Zubereitungen, die Amine der Formel I oder ihre Säureadditionssalze enthalten:

Beispiel A: Tabletten

Ein Gemisch von 1 kg "P"-Dihydrochlorid, 4 kg Lactose, 1,2 kg Kartoffelstärke, 0,2 kg Talk und 0,1 kg Magnesiumstearat wird in üblicher Weise zu Tabletten verpreßt, derart, daß jede Tablette 10 mg Wirkstoff enthält.

Beispiel B: Dragees

Analog Beispiel A werden Tabletten gepreßt, die anschließend in üblicher Weise mit einem Überzug aus Saccharose, Kartoffelstärke, Talk, Tragant und Farbstoff überzogen werden.

Beispiel C: Kapseln

2 kg "P"-Dihydrochlorid werden in üblicher Weise in Hartgelatinekapseln gefüllt, so daß jede Kapsel 20 mg des Wirkstoffs enthält.

Beispiel D: Ampullen

Eine Lösung von 1 kg "P"-Dihydrochlorid in 60 l zweifach destilliertem Wasser wird steril filtriert, in Ampullen abgefüllt, unter sterilen Bedingungen lyophilisiert und steril verschlossen. Jede Ampulle enthält 10 mg Wirkstoff.

Analog sind Tabletten, Dragees, Kapseln und Ampullen erhältlich, die einen oder mehrere der übrigen Wirkstoffe der Formel I und/oder ihre physiologisch unbedenklichen Säureadditionssalze enthalten.

**Patentansprüche**

1. Pyridinderivate der Formel I

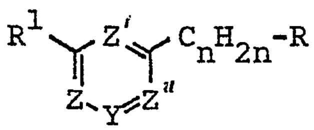

I

worin eine der Gruppen Y, Z, Z' und Z" N, die anderen dieser Gruppen jeweils CH, n 1, 2 oder 3,

R

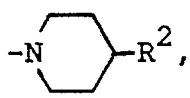

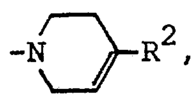

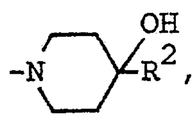

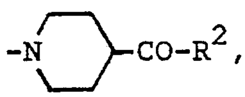

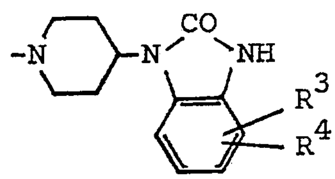

oder

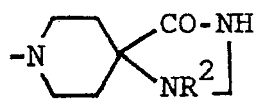

,

$R^1$ u. $R^2$ jeweils unsubstituierte oder ein- oder zweifach durch Alkyl und/oder Alkoxy mit jeweils 1-4 C-Atomen, F, Cl, Br, OH und/oder $CF_3$ substituierte Phenyl- oder 2- oder 3-Thienylreste und

$R^3$ u. $R^4$ jeweils H, Alkyl oder Alkoxy mit jeweils 1-4 C-Atomen, F, Cl, Br, OH oder $CF_3$

bedeuten,

wobei jedoch Y CH bedeutet, wenn R

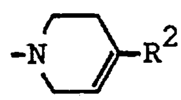

bedeutet, sowie ihre Salze.

2. a) 2-(4-Phenyl-1,2,3,6-tetrahydropyridyl-methyl)-4-phenylpyridin;
b) 3-Phenyl-5-(4-phenyl-piperidinomethyl)-pyridin.

3. Verfahren zur Herstellung von Pyridinderivaten der Formel I gemäss Anspruch 1 sowie von deren Salzen, dadurch gekennzeichnet, daß man eine Verbindung der Formel II

Py-A-$X^1_m$ II

worin Py den Rest

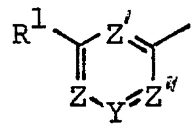

A die Gruppe $-C_nH_{2n}-$,

$X^1$ X oder $NH_2$,

X Cl, Br, J, OH oder eine reaktionsfähig funktionell abgewandelte OH-Gruppe bedeuten und

$R^1$, Y, Z, Z', Z" und n die angegebenen Bedeutungen haben,

mit einer Verbindung der Formel III

$X^2-CH_2CH_2-G-CH_2-X^3$ III

worin

$X^2$ u $X^3$ gleich oder verschieden sind und, falls $X^1 = NH_2$ ist, jeweils X, andernfalls zusammen NH und

G-$CHR^2-CH_2$-, $-CR^2=CH-$, $-CR^2(OH)-CH_2-$,

G $-CHR^2-CH_2-$, $-CR^2=CH-$, $-CR^2(OH)-CH_2-$,

$-CH(CO-R^2)-CH_2-$, 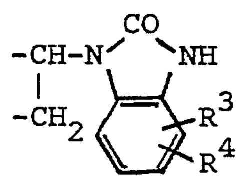 oder

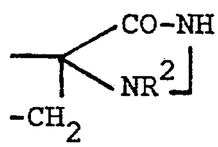

bedeuten und $R^2$, $R^3$ und $R^4$ die angegebenen Bedeutungen haben, umsetzt

oder daß man eine sonst der Formel I entsprechende Verbindung, die jedoch an Stelle eines oder mehrerer Wasserstoffatome eine oder mehrere reduzierbare Gruppen und/oder eine oder mehrere zusätzliche C-C- und/oder C-N-Bindungen enthält, mit einem reduzierenden Mittel behandelt

oder daß man zur Herstellung einer Verbindung der Formel I, worin

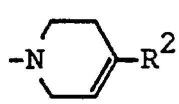

ist, eine Verbindung der Formel IV

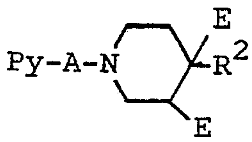

IV

worin

der eine Rest E X, CN oder $NH_2$,
der andere Rest E H bedeutet und
Py, A, $R^2$ und X die angegebenen Bedeutungen haben

mit einem HE-abspaltenden Mittel behandelt

und/oder daß man gegebenenfalls in einer Verbindung der Formel I eine O-Alkylgruppe unter Bildung einer OH-Gruppe spaltet

und/oder daß man eine Base der Formel I durch Behandeln mit einer Säure in eines ihrer Salze umwandelt.

4. Verfahren zur Herstellung pharmazeutischer Zubereitungen, dadurch gekennzeichnet, daß man eine Verbindung der Formel I gemäss Anspruch 1 und/oder eines ihrer physiologisch unbedenklichen Salze zusammen mit mindestens einem festen, flüssigen oder halbflüssigen Träger- oder Hilfsstoff und gegebenenfalls in Kombination mit einem oder mehreren weiteren Wirkstoff(en) in eine geeignete Dosierungsform bringt.

5. Pharmazeutische Zubereitung, gekennzeichnet durch einen Gehalt an mindestens einer Verbindung der Formel I gemäss Anspruch 1 und/oder einem ihrer physiologisch unbedenklichen Salze.

6. Verbindungen der Formel I gemäss Anspruch 1 und/oder ihre physiologisch unbedenklichen Salze zur Bekämpfung von Krankheiten.

7. Verwendung von Verbindungen der Formel I gemäss Anspruch 1 und/oder ihren physiologisch unbedenklichen Salzen zur Herstellung von Arzneimitteln.

8. Verbindungen der Formel V

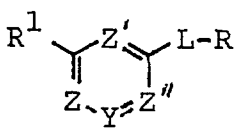

V

worin

R, $R^1$, $R^2$, Y und Z die bei Formel I gemäss Anspruch 1 angegebene Bedeutung haben und

L eine dem Rest $C_nH_{2n}$ entsprechende Kette bedeutet, worin jedoch eine oder mehrere $-CH_2$-Gruppe(n) durch -CO-Gruppe(n) und/oder ein oder mehrere Wasserstoffatome(e) durch OH-Gruppe(n) ersetzt sind.

## Claims

1. Pyridine derivatives of the formula I

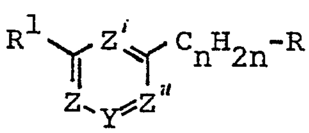

I

wherein one of the groups Y, Z, Z′ and Z″ is N and the other of these groups is in each case CH,
n is 1, 2 or 3,

R is 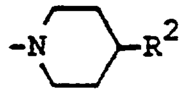, 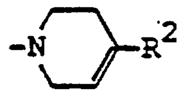, 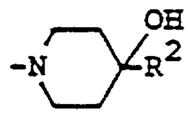,

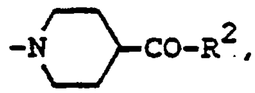, 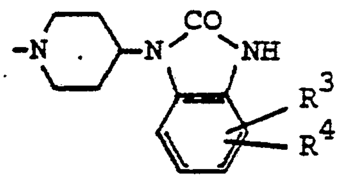

or 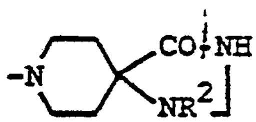,

$R^1$ and $R^2$ are in each case phenyl or 2- or 3-thienyl radicals which are unsubstituted or mono- or disubstituted by alkyl and/or alkoxy with in each case 1–4 C atoms, F, Cl, Br, OH and/or $CF_3$ and
$R^3$ and $R^4$ are in each case H, alkyl or alkoxy with in each case 1–4 C atoms, F, Cl, Br, OH or $CF_3$, but wherein Y is CH if R is

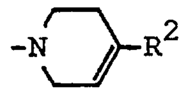

and their salts.

2. a) 2-(4-Phenyl-1,2,3,6-tetrahydropyridyl-methyl)-4-phenylpyridine;
b) 3-Phenyl-5-(4-phenyl-piperidinomethyl)-pyridine.

3. Process for the preparation of pyridine derivatives of the formula I according to Claim 1 and of salts thereof, characterized in that a compound of the formula II

$Py\text{-}A\text{-}X^1$ II

wherein
Py is the radical

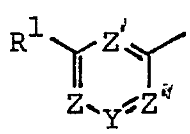

A is the group $-C_nH_{2n}-$,
$X^1$ is X or $NH_2$,
X is Cl, Br, I, OH or a reactively functionally modified OH group and
$R^1$, Y, Z, Z′, Z″ and n have the meanings given, is reacted with a compound of the formula III

$X^2\text{-}CH_2CH_2\text{-}G\text{-}CH_2\text{-}X^3$ III

wherein
$X^2$ and $X^3$ are identical or different and, if $X^1$ is $NH_2$, are in each case X, or otherwise together are NH and

G is $-CHR^2-CH_2-$, $-CR^2=CH-$, $-CR^2(OH)-CH_2-$,

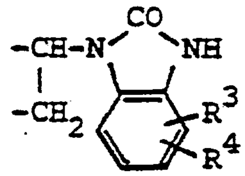

or

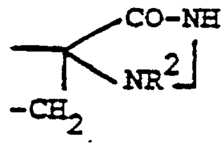

and
$R^2$, $R^3$
and $R^4$ have the meanings given,
or in that a compound which otherwise corresponds to the formula I but contains one or more reducible groups and/or one or more additional C–C and/or C–N bonds instead of one or more hydrogen atoms is treated with a reducing agent, or in that, to prepare a compound of the fomrula I
wherein R is

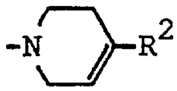

a compound of the formula IV

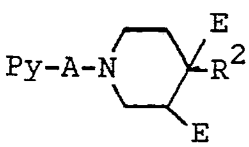

IV

wherein
one of the radicals E is X, CN or $NH_2$,
the other radical E is H and
Py, A, $R^2$ and X have the meanings given,
is treated with an agent which splits off HE, and/or in that, if appropriate, an O-alkyl group in a compound of the formula I is split to form an OH group, and/or in that a base of the formula I is converted into one of its salts by treatment with an acid.

4. Process for the preparation of pharmaceutical formulations, characterized in that a compound of the formula I according to Claim 1 and/or one of it physiologically acceptable salts is brought into a suitable dosage form together with at least one solid, liquid or semi-liquid excipient or auxiliary and if appropriate in combination with one or more other active compound(s).

5. Pharmaceutical formulation, characterized in that it contains at least one compound of the formula I according to Claim 1 and/or one of its physiologically acceptable salts.

6. Compounds of the formula I according to Claim 1 and/or their physiologically acceptable salts for combating diseases.

7. Use of compounds of the formula I according to Claim 1 and/or their physiologically acceptable salts for the preparation of medicaments.

8. Compounds of the formula V

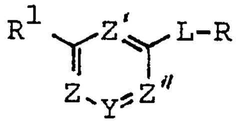

V

wherein
R, $R^1$, $R^2$, Y, Z, Z′ and Z″ have the meaning given in the case of formula 1 according to Claim 1 and

L is a chain corresponding to the radical $C_nH_{2n}$, but wherein one or more $-CH_2-$ group(s) are replaced by –CO– group(s) and/or one or more hydrogen atom(s) are replaced by OH group(s).

## Revendications

1. Dérivés de la pyridine de formule I

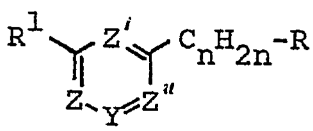

I

dans laquelle l'un des symboles Y, Z, Z′ et Z″ représente N, et les autres représentent chacun CH,
n est égal à 1, 2 ou 3,
R représente

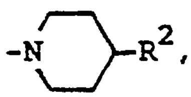, 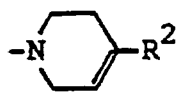, 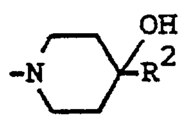,

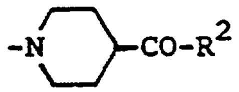, 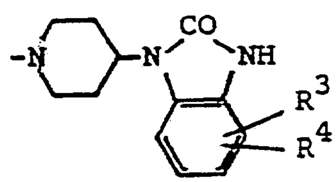

ou 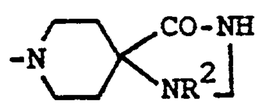,

$R^1$ et $R^2$ représentent chacun un groupe phényle non substitué ou mono- ou di-substitué par des groupes alkyle et/ou alcoxy en C1–C4, F, Cl, Br, des groupes OH et/ou $CF_3$, ou un groupe 2- ou 3-thiényle, et
$R^3$ et $R^4$ représentent chacun H, un groupe alkyle ou alcoxy en C1–C4, F, Cl, Br, un groupe OH ou $CF^3$, sous réserve que Y doit représenter CH lorsque R représente

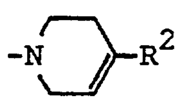

et leurs sels.

2. a) 1a 2-(4-phényl-1, 2, 3, 6-tétrahydropyridyl-méthyl)-4-phénylpyridine ;
b) 1a 3-phényl-5-(4-phényl-pipéridinométhyl)-pyridine.

3. Procédé de préparation des dérivés de la pyridine répondant à la formule I de la revendication 1 et de leurs sels, caractérisé en ce que l'on fait réagir un composé de formule II

Py-A-$X^1$ II

dans laquelle
Py représente le groupe

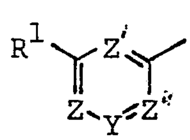

A représente un groupe $-C_nH_{2n}-$,
$X^1$ représente X ou $NH_2$,
X représente Cl, Br, I, un groupe OH ou un groupe OH à l'état de dérivé fonctionnel réactif, et
R1, Y, Z, Z', Z" et n ont les significations indiquées ci-dessus,
avec un composé de formule III

$X^2-CH_2CH_2-G-CH_2-X^3$ III

dans laquelle $X^2$ et $X^3$, ayant des significations identiques ou différentes, représentent chacun X lorsque $X^1 = NH_2$ et forment ensemble, dans les autres cas, le groupe NH, et G représente $-CHR^2-CH_2-$, $-CR^2=CH-$, $-CR^2(OH)-CH_2-$, $-CH(CO-R^2)-CH_2-$,

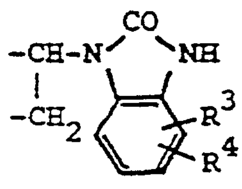

ou

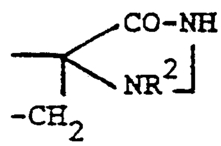

$R^2$, $R^3$ et $R^4$ ont les significations indiquées ci-dessus, ou bien en ce que l'on traite par un agent réducteur un composé répondant par ailleurs à la formule I mais contenant, à la place d'un ou plusieurs atomes d'hydrogène un ou plusieurs groupes réductibles et/ou une ou plusieurs liaisons supplémentaires C–C et/ou C–N, ou bien en ce que, pour préparer un composé de formule I dans laquelle R représente

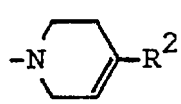

on traite un composé de formule IV

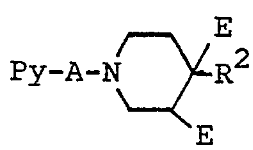

dans laquelle l'un des symboles E représente X, CN ou $NH_2$, l'autre représente H, et Py, A, $R^2$ et X ont les significations indiquées ci-dessus, par un agent provoquant la scission de HE, et/ou en ce que le cas échéant, dans un composé de formule I, on scinde un groupe O-alkyle avec formation d'un groupe OH, et/ou en ce que l'on convertit une base de formule I, par traitement à l'aide d'un acide, en l'un de ses sels.

4. Procédé de préparation de compositons pharmaceutiques, caractérisé en ce que l'on met sous une forme de dosage approprié un composé de formule I de la revendication 1 et/ou un sel d'un tel composé acceptable pour l'usage pharmaceutique, avec au moins un véhicule ou produit auxiliaire solide, liquide ou semi-liquide et le cas échéant en combinaison avec une ou plusieurs autres substances actives.

5. Composition pharmaceutique, caractérisée en ce qu'elle contient au moins un composé de formule I de la revendication 1 et/ou un sel d'un tel composé acceptable pour l'usage pharmaceutique.

6.Composés de formule I de la revendication 1 et/ou leurs sels acceptables pour l'usage pharmaceutique, pour le traitement de maladies.

7. Utilisation des composés de formule I de la revendication 1 et/ou de leurs sels acceptables pour l'usage pharmaceutique, pour la préparation de médicaments.

8. Composés de formule V

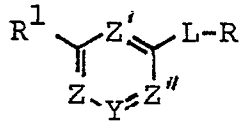

dans laquelle R, $R^1$, R2, Y, Z, Z' et Z" ont les significations indiquées en référence à la formule I, et L représente une chaîne correspondant au groupe $C_nH_{2n}$ mais dans laquelle toutefois ua ou plusieurs groupes -CH2- ont été remplacés par des groupes -CO- et/ou un ou plusieurs atomes d'hydrogène ont été remplacés par des groupes OH.